# EUROPEAN PATENT APPLICATION

(11) **EP 4 501 392 A1**
(43) Date of publication of application: **05.02.2025**
(21) Application number: 23188795.1
(22) Date of filing: 31.07.2023
(51) Int. Cl.: A61M 60/196, A61M 60/258, A61M 60/441, A61M 60/531, A61M 60/546, A61M 60/554, A61M 60/835

(54) **CONTROL DEVICE FOR A HEART PUMP**

(71) Applicant: Charité - Universitätsmedizin Berlin, 10117 Berlin (DE)
(72) Inventor: The designation of the inventor has not yet been filed
(74) Representative: Gulde & Partner

(57) **Abstract**

The present invention relates to control device (1) for a heart pump (2) of an artificial heart (3) with a right pumping chamber (4) and with a left pumping chamber (5), which is connectable to a native left atrium and to a native right atrium of a biological heart, with a device (8) for determining a central venous pressure and/or an average pressure at least in one atrium, in particular the right atrium, with a device (9) for assigning an operating parameter, in particular the delivery rate of the heart pump (2), further in particular a pump rate, speed or an electric pump power..

A control device (2) for a total artificial heart (4), which normalizes the patients' hemodynamic condition and adapts the output to the patients need with a minimum number of sensors, is characterized in that, there is a fluidic connection device to establish a fluidic connection (10) between the left atrium and the right atrium.

## Description

Control device for a heart pump of an artificial heart with a right pumping chamber and with a left pumping chamber, which is connectable to at least a part of a native left atrium and at least to a part of a native right atrium of a biological heart, with a device for determining a mean central venous pressure and/or a mean pressure at least in one atrium, in particular the right atrium and/or an end-diastolic filling pressure, with a device for assigning an operating parameter, in particular the delivery rate of the heart pump, further in particular a pump rate, speed or an electric pump power.

In addition, the invention relates to a method for operating a control device for a heart pump of an artificial heart.

Furthermore, the invention relates to a computer program product and a heart pump or heart pump system comprising a control device.

The present invention also relates to a total artificial heart with a right ventricle pumping chamber and with a left ventricle pumping chamber, which is connectable to a native left atrium and to a native right atrium of a biological heart, and with a heart pump.

For patients with incurable heart disease, artificial hearts are an alternative to transplantation of a natural heart, mainly due to the lack of donors and long waiting times. There are so-called ventricular assist devices that can be implanted in addition to the patient's natural heart to support it in cases of temporary cardiac insufficiency where recovery of the heart or transplantation of a natural heart from a donor is foreseeable. Furthermore, attempts have been made to create artificial hearts to completely replace a patient's heart (Total Artificial Heart).

Such systems have to meet special requirements in terms of reliability, because failure of the artificial heart would result in the immediate death of the patient. In this respect, the task of creating an artificial heart that can be operated as long as possible, reliably, with low wear and without malfunctions represents a particular challenge.

Mechanical circulatory support (MCS) became the therapy of choice for end-stage heart failure patients who are not eligible for heart transplantation. Currently, ventricular assist devices (VADs) account for the vast majority of all MCS implantations, outnumbering heart replacements with total artificial hearts (TAHs) with only 200 TAH implantations in the era 2014-2018 in the US. However, these low numbers rather reflect the limited availability of devices than the medical need.

Emerging patient populations in need of a TAH are patients with e.g. cardiac tumors, biventricular failure, congenital heart disease or univentricular physiologies. In all these patients conventional VADs are either not applicable or lead to worse clinical outcomes in terms of survival than in typical VAD patients with left ventricular failure.

The low TAH implantation numbers may be limited by intrinsic disadvantages of their underlying pumping principle. The available displacement devices, mimicking the native pulsatile heart function, are of large size and limited durability. Which is mainly attributed to the use of membranes and artificial mechanical valves. Compared to state-of-the-art VADs. The peripherals are larger with limited battery life. The technology of Rotary Blood Pumps (RBPs) permits small and efficient pump designs. However, this pumping principle is associated with non-physiologic continuous flow, high shear rates, which are suspected to induce blood trauma mechanisms associated with adverse events such as gastrointestinal bleeding, strokes, and pump thrombus formation.

Rotary piston pumps offer a pumping principle which may have the potential to overcome limitations of the current technology: They may be smaller, more durable (no membrane and valves) and more efficient than pulsatile device and concomitantly provide pulsatile flow with a potentially less traumatic pumping principle than rotary blood pumps. However. their actuation and bearing concepts are usually complex. Theoretical derivations suggest that, if the development of reliable drives and bearings for this pump technology succeeds, rotary piston blood pumps may become a promising alternative. First innovative blood pump concepts inspired by rotary piston pumps indicated the technical feasibility and the preclinical potential of such approaches. Yet, none of these devices has been translated into the clinical arena. We previously disclosed a TAH based on an innovative pumping concept, which combines the advantages of both pulsatile and rotary blood pumps - pulsatile flow propulsion at a low blood trauma and low technical complexity with a minimum number of moving parts.

However, all described and novel TAH systems rely either on a passive system to adapt the output of the left and right pump and prevent venous pulmonary congestion or an active system relying on a multitude of sensors to adapt the output of the left and right pump.

Whereas the adaption of cardiac output to the patients need is limited with the passive system. With the active system the multitude of sensors introduces a high risk of failure and limited durability.

The objective of the present invention is based on the task to develop a physiologic control system for a total artificial heart, which normalizes the patients' hemodynamic condition and adapts the output to the patients need with a minimum number of sensors.

In the present invention, this task is solved by the features of the identification part of patent claim 1, first of all, in that there is a fluidic connection device to establish a fluidic connection between the left atrium and the right atrium. This can be realized by either a connection between the native atria or between pump components ("grafts") connecting the pump to the native atria.

The total artificial heart aims to replace a patient's diseased heart. Therefore, the artificial heart also includes a left and a right pumping chamber, replacing the native ventricles. In the present invention, it is preferred that the grafts can be patched to at least a part of the left and right atrium of a biological heart.

In this way, the grafts can also be sutured to the atriae with a connection between the left and right atrial graft. However, it is also conceivable that the connection is sutured to still native atrial remnants. In this case, the ventricles are cut above the valve plane, leaving a portion of the atria, which are, however, approximately half open due to the cutting. By means of a comparatively large patch, the artificial heart can then be connected to the biological part.

The fluidic connection can be established in the remaining biological part by the device for establishing a fluidic connection. Preferably, however, this connection is formed in the mechanical, constructive part. In this way, unnecessary surgical intervention in the biological part can be avoided, since the fluidic connection is formed directly in the mechanical part.

Accordingly, the aforementioned devices may also be included in the artificial part of the heart.

The end-diastolic filling pressure is the pressure within the, preferably right, ventricle following the completion of diastolic filling, just prior to systole.

The mean central venous pressure is the average pressure in the thoracic vena cava near the right atrium. The mean central venous pressure is an important factor in critical care medicine because it can be used to estimate a patient's fluid volume status, assess cardiac function, and gauge how well the right ventricle of the heart is functioning.

The atrial pressure, especially the right atrial pressure, is the blood pressure in the (right) atrium of the heart. The right atrial pressure reflects the amount of blood returning to the heart and the ability of the heart to pump the blood into the arterial system. The right atrial pressure is often nearly identical to central venous pressure, although the two terms are not identical, as a pressure differential can sometimes exist between the venae cavae and the right atrium. The central venous pressure and the right atrial pressure can differ when venous tone (i.e the degree of venous constriction) is altered.

The mean central venous pressure and the mean atrial pressure can be calculated via a moving average filter of the currently measured central venous pressure and atrial pressure, respectively, whereas the averaging time window is a whole multiple of the current cycle time of the pump, with 1 to 10 multiples of the cycle times, most preferably 1 multiples of the cycle time.

The mean average values can be calculated using an integration of the corresponding currently measured pressure followed by a triggered division of the integrated time steps. The trigger is activated after 1 to 10 cycle times, most preferably after 1 cycle time.

The cycle time is the time the pump takes for one cycle form systole to diastole, which is reciprocal to the pump rate.

Of course, these values can also be determined using other common suitable methods. The above procedures are listed as examples.

An operating parameter of the heart pump can be understood to mean various variables, which can be influenced and which can be measured or adjusted during operation of the heart pump. These include factors that influence the operating sequence and function of the heart pump. These include load characteristics, dynamic behaviour, installation conditions, operating conditions and so on, as well as adjustable parameters such as speeds, flow rates, pressures and comparable variables.

This physiologic control system for a total artificial heart balances the left and right side in a passive manner. This has the advantage of a low complexity and a high reliability compared to active systems. Furthermore, it requires only a single hemodynamic sensor. This also has the advantage of low complexity and high reliability compared to systems relying on multiple sensors to adapt the pump to the need of the patient in daily life during rest and exercise which leads to an enhanced quality of life.

In a first embodiment of the control device according to the invention, it is provided that the fluidic connection is realized by means of connectors, stents, or cannulas. By means of this constructional design, the fluidic connection can be realized in a simple manner through already existing structures or uniform structures of the entire artificial heart, meaning the type of grafts etc. can be used that is already used for other connections of the artificial heart and/or the heart pump. It is not necessary to develop a new connection design.

In a further advantageous embodiment of the control device according to the invention, it is provided that the fluidic connection has a diameter in a range between 4 mm and 12 mm, preferably between 6 mm and 10 mm, particularly preferably a diameter of 8 mm. It can be observed that hemodynamics during variation of atrial shunt resistance at a constant frequency of the heart pump, constant hemodynamic conditions and constant bronchial shunt (10 mmHg*s/ml) a resistance below 0.1 mmHg*s/ml, which corresponds to a communication diameter of approximately 8mm, leads to a pressure difference of less than 1 mmHg. Therefore, a diameter of 8 mm or more is particularly advantageous.

The present invention can be used for any suitable type of pump, that is suitable to function as a heart pump. In a particularly preferred embodiment of the control device according to the invention, the heart pump is designed as a rotary piston pump. Rotating piston pumps have the advantage that, despite rotating parts, they generate a pulsating flow that can well simulate the pumping performance of a real heart. In addition, there is no need for diaphragms or valves, which are maintenance-prone and could cause the pump to malfunction.

Furthermore, in another embodiment of the control device according to the invention, it can be provided that the heart pump comprises a controller, that the controller comprises at least one memory, and that at least presets for operating parameters of the heart pump can be stored in the memory. In this way, the heart pump can be given parameters under which the heart pump is to operate. It is also conceivable that different limit values are stored, at whose interval the heart pump is to operate.

The controller can be programmed via different ways. Preferably, a wireless interface is used. Conceivable would be a transmission via a Uu link via mobile radio. In addition, a Wifi-based standard such as IEEE 802.11p via direct communication or also Bluetooth protocols can also be used.

Furthermore, in an advantageous embodiment of the control device according to the invention, it can be provided that a control signal can be transmitted to the device for assigning the operating parameter by the device for determining a mean central venous pressure and/or a mean atrial pressure and/or an end-diastolic filling pressure, wherein an operating parameter of the heart pump can be set by the device for assigning the operating parameter as a function of the mean central venous pressure and/or a mean atrial pressure and/or a an end-diastolic filling pressure. In this way, the heart pump can respond to changes induced due to physical circumstances of a patient. The pump rate, speed or power of the pump can then be solely dependent on the actual requirement of the patient, so that supply to the patient is guaranteed in a wide variety of situations.

The aforementioned task is further solved by a method for operating a control device according to the invention for a heart pump of an artificial heart. It is provided that an operating parameter of the heart pump is adjusted in response to a change in the mean central venous pressure and/or the mean atrial pressure and/or end-diastolic filling pressure at least in one atrium. The above explanations concerning the control device according to the invention also apply accordingly to the method according to the invention.

In a first embodiment of the method according to the invention, it is provided that an initial setting of the heart pump is carried out, with a predetermined mean central venous pressure and/or an mean atrial pressure and/or end-diastolic filling pressure being set at least in one atrium during a setting. This setting can be made by medical personnel, for example, whereby a mean central venous pressure or mean atrial pressure and/or end-diastolic filling pressure can be set on the basis of various examinations of the patient. In this way, a basic setting tailored to the patient can be found that initially provides the patient with the best possible blood supply.

In a further embodiment of the method according to the invention, it is provided that the control device is operated in a physiological control mode and that an increase or a decrease in the mean atrial pressure compared to the set mean atrial pressure leads to an increase or decrease, respectively, in an operating parameter of the heart pump. In this way, it is possible to respond automatically to a change in the patient's requirement. A deviation of the actual pressure from the comparison value can be detected by a comparison value, which is found, for example, in the basic settings, which causes a reaction of the adjustment of the operating parameter of the pump. Accordingly, the adjustment is made in such a way that a value for the mean central venous pressure or mean atrial pressure is found which is optimal for the patient in the corresponding situation. These adjustments can therefore be made both proportionally and anti proportionally.

In a preferred embodiment of the method according to the invention, it is provided that a proportionality factor Kp is defined and that a variable sensitivity of an adaptation of the operating parameter of the heart pump to a change in the mean central venous pressure and/or the mean atrial pressure and/or end-diastolic filling pressure at least in one atrium is realized by the proportionality factor Kp. It is also conceivable that different proportionality factors are defined for different intervals of the mean central venous pressure and/or the mean atrial pressure and/or end-diastolic filling pressure, so that the extent of the response of the heart pump is correspondingly larger or smaller, depending on the range in which one is located. In higher, more extreme ranges, it may be possible for the response to be more direct in order to quickly return pressures or return flows to an appropriate level. In the case of small deviations from the setpoint, for example a setpoint found in the basic settings, smaller, gentler adjustments are sufficient to avoid unpleasant physical reactions.

Furthermore, in a further embodiment of the method according to the invention, it can be provided that an estimate of a mean aortic pressure is made by means of the power applied by the pump during a systole. The term systole refers to one of the two cardiac phases during the pumping movements of the heart. During systole, the heart contracts, thereby pumping blood into the systemic circulation. The relaxation phase, on the other hand, is called diastole. Both phases take place alternately. During systole, it can be assumed that there is an adequate mean aortic pressure, whereas fluctuations can occur during the transition from systole to diastole.

The aforementioned task is also solved by a computer program product with a program that calculates and assigns an operating parameter of a heart pump to a determined mean atrial pressure in an atrium and/or a mean central venous pressure and/or end-diastolic filling pressure and controls the heart pump with this operating parameter. The computer program product can of course also be designed in such a way that it implements the aforementioned method for controlling a heart pump.

The aforementioned task is also solved by a heart pump containing a control device according to the invention. The above explanations concerning the control device according to the invention also apply accordingly to the heart pump or heart pump system according to the invention.

The aforementioned task is also solved by a total artificial heart with a right ventricle pumping chamber and with a left ventricle pumping chamber, which is connectable to a native left atrium and to a native right atrium of a biological heart, and with a heart pump. It is envisaged that the heart pump is designed according to the invention. The above explanations concerning the heart pump according to the invention also apply accordingly to the total artificial heart according to the invention.

It may further be provided that the heart pump or heart pump system is set up for control by a computer program product according to the invention and/or set up for operation of a method according to the invention.

Electronic or electrical devices and/or other relevant devices or components according to embodiments of the present invention described herein may be implemented using any suitable hardware, firmware (e.g., an application-specific integrated circuit), software, or a combination of software, firmware, and hardware. For example, the various components of these devices may be located on an integrated circuit (IC) or on separate IC chips. In addition, the various components of these devices may be implemented on a flexible printed circuit film, a tape carrier package (TCP), a printed circuit board (PCB), or on a single substrate. Further, the various components of these devices may be a process or thread that runs on one or more processors in one or more computing devices, executes computer program instructions, and interacts with other system components to perform the various functions described herein. The computer program instructions are stored in a memory that may be implemented in a computing device using a standard memory, such as random access memory (RAM).

The computer program instructions may also be stored in other non-transferable computer-readable media, such as a CD-ROM, flash drive, or the like. A person skilled in the art should also recognize that the functionality of various computing devices may be combined or integrated into a single computing device, or that the functionality of a particular computing device may be distributed to one or more other computing devices, without departing from the scope of the exemplary embodiments of the present invention.

The various embodiments of the invention mentioned in this application can be advantageously combined with each other, unless otherwise specified in the individual case.

The invention is explained below in embodiment examples with reference to the associated drawings. It shows:
- Figure 1: A schematic representation of a control device for a heart pump,
- Figure 2: Hemodynamics during variation of atrial shunt resistance,
- Figure 3: A schematic representation of the operation of the control device accord-ing to figure 1, and
- Figure 4: Hemodynamics during preload variations at a set pump frequency.

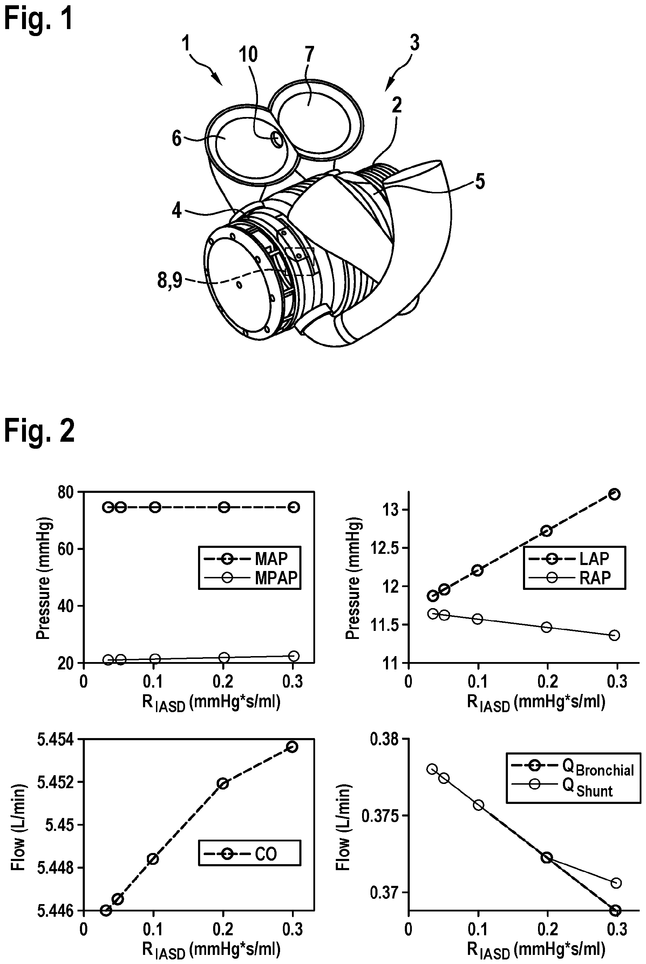

Fig. 1 shows a control device 1 for a heart pump 2 of an artificial heart 3 with a right ventricle 4 and with a left ventricle 5, and with a left atrium 6 and with a right atrium 7. Further comprising a device 8 for determining a mean central venous pressure and an average atrial pressure in the right atrium 7. In addition, a device 9 for assigning an operating parameter of the heart pump 2 is provided. The operating parameter is a delivery rate of the heart pump or an electrical pump output. In addition, a fluidic connection 10 is formed between the left atrium 6 and the right atrium 7.

The cardiac output of a left ventricle 5 and right ventricle 4 in a healthy human is slightly different, due to the bronchial shunt the cardiac output of the left ventricle is up to 15% higher than the one of the right ventricle. Additionally, in circulatory failure, collaterals and shunts may lead to imbalance of the left and right cardiac output. Further, in the heart pump 2, a gap-flow from the left to the right chamber introduces an additional source of imbalance of left and right cardiac output.

The fluidic connection 10 between the left atrium 6 and the right atrium 7 balances the atrial pressures in case of an imbalanced cardiac output: The larger the communication, the lower its resistance and the less the difference between left and right atrial pressure.

Figure 2 shows hemodynamics during variation of atrial shunt resistance R_{IASD} a constant heart pump 2 frequency at 3.4 Hz, constant hemodynamic conditions and constant bronchial shunt at 10 mmHg*s/ml. It can be observed that a resistance below 0.1 mmHg*s/ml, which corresponds to a fluidic connection 10 diameter of approximately 8 mm, leads to a pressure difference of less than 1 mmHg. MAP in this context means arterial pressure; MPAP is the pulmonary arterial pressure; LAP stands for left atrial pressure; and RAP for right atrial pressure. CO is the Cardiac Output, wherein Q_{Bronchial} stands for the flow across the bronchial shunt. Q_{Shunt} is the flow across the interatrial communication.

Although a communication between the atriae is often considered a risk for Cross-thrombi from the venous to arterial side, recent studies indicated the safety of such a therapeutic approach in heart failure patients with preserved ejection fraction (https://ziiwwwncbi.nlm.nih.govipmciarticlesiPMC6233816/). These results underpin the potential of this simple approach to balance left and right cardiac output also in TAH patients.

This passive system to balance the left and right cardiac output is combined with an active physiologic control system, which adapts the heart pumps 2 output based on measurements of a sensor in the right atrium or central veins.

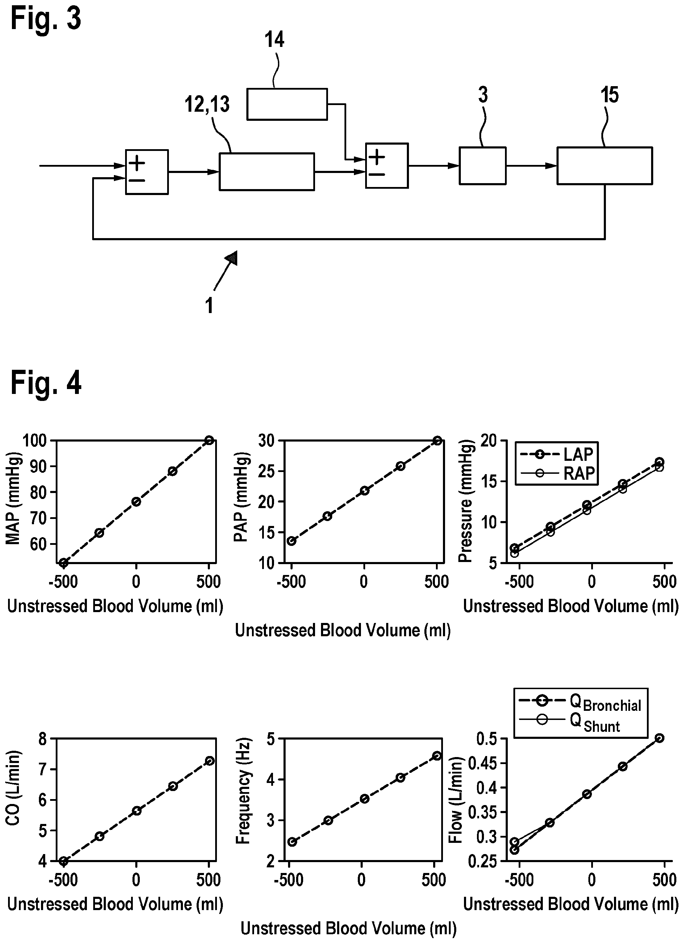

Figure 3 shows a schematic illustration of the control strategy. The control strategy is based on an initial condition which is defined by healthcare personal for each patient individually: E. g. for the heart pump 2 the pump rate or frequency will be adjusted until a desired right atrial and/or central venous pressure is achieved. This setting is stored within a memory 13 of a controller 12 of the heart pump. After switching into a physiologic control mode each increase or decrease of right atrial or central venous pressure compared to the initial set value will lead to an automatic increase or decrease in pump rate and consequently the output of the heart pump 2. The current strategy relies on a simple proportional control, which proportional value Kp defines the preload sensitivity of the control system, meaning how much increase of pump output for a certain difference of set and measured right atrial or central venous pressure is necessary. Figure 3 indicates a schematic of the control loop. Various known controllers can be used for the control. Since there is a proportional relationship, the use of a P-controller is beneficial. As can be seen in Figure 3 a baseline frequency 14 is deployed into the system, whereas the artificial heart can pump the blood into the cardiovascular system 15.

Figure 4 shows a simulation of pumps and hemodynamic response to changes in venous pressure. This is induced by adaptation of the unstressed venous blood volume. An increase in venous pressure results in elevated RAPs, which in turn leads to an increase of the rate or frequency of the heart pump.

Figure 4 shows hemodynamics during preload variations at a set frequency of the heart pump at 3 Hz and a set RAP of 12.5 mmHg at constant hemodynamic conditions, constant bronchial and atrial shunts. A linear increase of the frequency and consequently CO can be observed with increase at venous pressure.

These results indicate that a physiologic control system based on a combination of passive and active features can effectively balance the left and right cardiac output and concurrently adapt the heart pumps 2 output to the demand of the patient.

Of note, this control strategy is theoretically independent of afterload pressures (systemic and pulmonary arterial pressures). To prevent excessive hypertension in the arterial systems, this control strategy may be completed by an estimate of the afterload pressure based on the power/current consumption of the heart pump during systole. Based on the estimated safe regions of pulmonary and arterial pressures the preload responsive physiologic control can be operated. In case of a hypo- or hypertensive condition which exceeds this redefined region, the control strategy can be overruled to reduce (hypertension) or increase (hypotension) cardiac output accordingly.

### List of reference signs

- 1.: Control device
- 2.: Heart pump
- 3.: Artificial Heart
- 4.: Right ventricle
- 5.: Left ventricle
- 6.: Left atrium
- 7.: Right atrium
- 8.: Device for determining a central venous pressure and/or an average atrial pressure
- 9.: Device for assigning an operating parameter
- 10.: Fluidic connection
- 11.: Diameter
- 12.: Controller
- 13.: Memory
- 14.: Baseline frequency
- 15.: Cardiovascular system

## Claims

1. Control device (1) for a heart pump (2) of an artificial heart (3) with a right pumping chamber (4) and with a left pumping chamber (5), which is connectable to at least a part of a native left atrium and at least to a part of a native right atrium of a biological heart,
with a device (8) for determining a mean central venous pressure and/or a mean atrial pressure and/or an end-diastolic filling pressure at least in one atrium, in particular the right atrium
with a device (9) for assigning an operating parameter, in particular the delivery rate of the heart pump (2), further in particular a pump rate, speed or an electric pump power, **characterized in that**,
there is a fluidic connection device to establish a fluidic connection (10) between the left atrium (6) and the right atrium (7).

2. Control device (1) according to claim 1, **characterized in that** the fluidic connection (10) is realized by means of connectors, stents or cannulas.

3. Control device (1) according to claim 1 or 2, **characterized in that** the fluidic connection (10) has a diameter (11) in a range between 4 mm and 12 mm, preferably between 6 mm and 10 mm, particularly preferably a diameter of 8 mm.

4. Control device (1) according to one of claims 1 to 3, **characterized in that** the heart pump (2) is designed as a rotary piston pump.

5. Control device (1) according to any one of claims 1 to 4, **characterized in that** the heart pump (2) comprises a controller (12), **in that** the controller comprises at least one memory (13), and **in that** at least presettings for operating parameters of the heart pump (2) can be stored in the memory (13).

6. Control device (1) according to one of claims 1 to 5, **characterized in that** a control signal can be transmitted by the device (8) for determining a mean central venous pressure and/or a mean atrial pressure and/or an end-diastolic filling pressure to the device (9) for assigning the operating parameter, wherein an operating parameter of the heart pump (2) can be set by the device (9) for assigning the operating parameter as a function of the mean central venous pressure and/or a mean atrial pressure and/or an end-diastolic filling pressure

7. A method of operating a control device (1) for a heart pump (2) of an artificial heart (3) according to any one of claims 1 to 6,
**characterized in that**
an operating parameter of the heart pump (2) is adjusted in response to a change in the mean central venous pressure and/or the mean atrial pressure at least in one atrium (6, 7) and/or an end-diastolic filling pressure

8. A method according to claim 7, **characterized in that** an initial setting of the heart pump (2) is made, wherein a predetermined mean central venous pressure and/or mean atrial pressure and/or an end-diastolic filling pressure is set in at least one atrium (6, 7) during a setting.

9. Method according to claim 8, **characterized in that** the control device is operated in a physiological control mode and **in that** an increase or a decrease, respectively, in the mean atrial pressure compared to the set mean atrial pressure leads to an increase or decrease, respectively, in an operating parameter of the heart pump (2).

10. Method according to any one of claims 7 to 9, **characterized in that** a proportionality factor Kp is defined and **in that** a variable sensitivity of an adaptation of the operating parameter of the heart pump (2) to a change in the mean central venous pressure and/or the mean atrial pressure at least in one atrium (6, 7) and/or an end-diastolic filling pressure is realized by the proportionality factor Kp.

11. A method according to any one of claims 7 to 9, **characterized in that** an estimate of a mean aortic pressure is made by means of the power applied by the heart pump (2) during systole.

12. Computer program product with a program which calculates and assigns an operating parameter of a heart pump (2) to a determined mean atrial pressure in an atrium (6, 7) and/or a mean central venous pressure and/or an end-diastolic filling pressure and controls the heart pump (2) with this operating parameter.

13. A heart pump (2) comprising a control device (1) according to any one of claims 1 to 6.

14. A heart pump (2) adapted to be controlled by a computer program product according to claim 13 and/or adapted to operate a method according to any one of claims 7 to 11.

15. A total artificial heart with a right ventricle pumping chamber (4) and with a left ventricle pumping chamber (5), which is connectable to a native left atrium and to a native right atrium of a biological heart, and with a heart pump according to any one of claims 13 or 14.
